(19) Europäisches Patentamt
European Patent Office
Office européen des brevets

(11) Publication number: **0 146 265 B1**

(12) **EUROPEAN PATENT SPECIFICATION**

(45) Date of publication of patent specification: **03.04.91**

(51) Int. Cl.[5]: **C12P 1/00**, C12P 21/02, C12P 25/00, C12P 19/32

(21) Application number: **84307942.7**

(22) Date of filing: **15.11.84**

(54) **Process for producing a compound from its precursor using an enzymatic activity of bacterium.**

(30) Priority: **15.11.83 JP 213319/83**
**21.09.84 JP 198280/84**

(43) Date of publication of application:
**26.06.85 Bulletin 85/26**

(45) Publication of the grant of the patent:
**03.04.91 Bulletin 91/14**

(84) Designated Contracting States:
**DE FR GB**

(56) References cited:
**EP-A- 0 084 975**

(73) Proprietor: **KYOWA HAKKO KOGYO CO., LTD.**
**6-1, Ohte-Machi Itchome**
**Chiyoda-ku Tokyo(JP)**

(72) Inventor: **Fujio, Tatsuro**
**6-29-1, Sagamidai**
**Sagamihara-shi Kanagawa-ken(JP)**
Inventor: **Hayashi, Mineyuki**
**3962-6, Ami-machi Oaza Ami**
**Inashiki-gun Ibaraki-ken(JP)**
Inventor: **Tomiyoshi, Yasuhiro**
**3-6-6, Asahi-cho**
**Machida-shi Tokyo(JP)**
Inventor: **Ozaki, Atsuko**
**3-9-15, Naka-machi**
**Machida-shi Tokyo(JP)**

(74) Representative: **Lambert, Hugh Richmond et al**
**D. YOUNG & CO. 10 Staple Inn**
**London, WC1V 7RD(GB)**

**EP 0 146 265 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a process for producing a compound producable from its precursor in the presence of adenosine-triphosphate (ATP) using enzymatic activity of bacteria. Such compounds include glutathione, guanosine-5'-phosphate (GMP), flavin-mononucleotide (FMN), $\gamma$-glutamylcysteine ($\gamma$-GC), glutamine and ATP.

Processes for producing such compounds using bacteria in a medium containing ATP are known. For example, glutathione is produced from glutathione precursors such as L-glutamic acid, L-cysteine and glycine according to the enzymatic reaction described below.

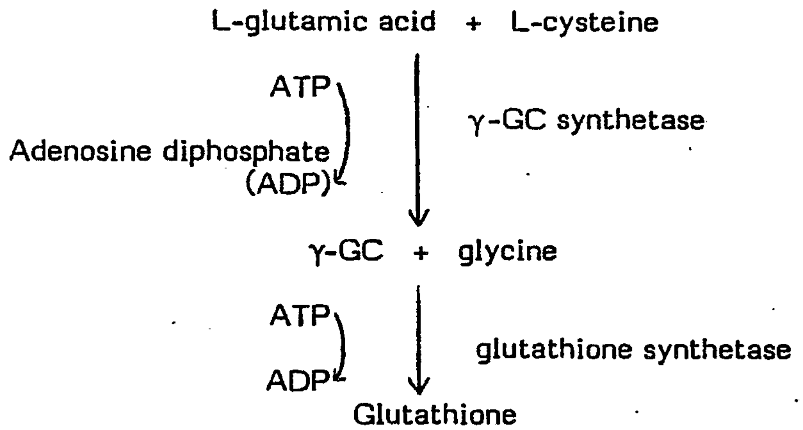


This reaction requires energy and two moles of ATP per one mole of formed glutathione are consumed to form two moles of ADP.

ATP is expensive and therefore, it is desired to develop a method for providing ATP at low cost or a method for reproducing ATP from ADP formed in the enzymatic reaction. It is known that phosphorylated compounds such as acetyl-phosphoric acid and carbamyl phosphate are used as source of energy donor. However,they are also expensive and are unstable.

As a result of studies on the method of reproducing ATP from ADP, etc. formed in the enzymatic reaction, it has been found that certain bacteria catalyze the enzymatic reaction in which the desired compound is produced from its precursor in the presence of ATP and have ATP reproducing system.

The ATP reproducing system means that the bacteria have an ability to convert ATP precursor such as ADP, adenosine monophosphate (AMP) or adenosine formed in the enzymatic reaction mixture, back into ATP in the presence of a non-phosphorylated compound as an energy donor.

In accordance with the present invention, therefore, there is provided a process for the production of a given compound by the enzymatic conversion of a precursor for that compound in the presence of ATP and which comprises contacting the precursor in an aqueous culture medium containing ATP with the microbial cells, or an enzymatically active component therefrom, of a bacteria having the ability to convert the said precursor into said compound in the presence of ATP, wherein said microbial cells, or said enzymatically active component, are or is derived from, respectively, a bacterium having the additional ability to produce ATP in situ in said medium from an ATP precursor present in said medium and in the presence of a non-phosphorylated energy donor, such energy donor likewise being present in said medium.

As the bacterium used in the present invention, any bacteria may be used so long as they have an ability to produce the desired compound from its precursor in the presence of ATP and have ATP reproducing system.

A process for producing glutathione according to the present invention is described below.

Any bacterium may be used so long as it has an ability to convert ADP to ATP in the presence of energy donor (E) and if necessary phosphate ion (P) and/or magnesium ion (Mg) and to produce glutathione in the presence of L-glutamic acid, L-cysteine and/or L-cystine and glycine.

Examples of the preferred strain include Escherichia coli ATCC 11303, Escherichia coli FERM BP-47, Escherichia coli FERM BP-48, Enterobacter aerogenes ATCC 13048, Proteus vulgaris FERM BP-655 and Erwinia herbicola ATCC 21434.

As the medium employed in the present process, any synthetic or natural medium can be employed so long as it contains a proper carbon source, a nitrogen source, inorganic materials and other nutrients necessary for the strain utilized.

As the carbon source, carbohydrates such as glucose, fructose, sucrose, maltose, mannose, galactose, xylose, etc., sugar alcohols such as sorbitol, mannitol, glycerol, etc., starch, starch hydrolyzate, molasses, etc., organic acids such as pyruvic acid, lactic acid, acetic acid, etc., amino acids such as aspartic acid, alanine, etc. may be used.

As the nitrogen source, ammonia, ammonium salts such as ammonium chloride, ammonium phosphate, ammonium sulfate, ammonium nitrate, ammonium carbonate ammonium acetate, etc., urea and other nitrogen containing materials such as peptone, NZ-amine, meat extract, yeast extract, corn steep liquor, casein hydrolyzate, fish meal or its digested product, soybean meal or its digested product, chrysalis hydrolyzate, etc., amino acids such as aspartic acid, glutamic acid, threonine, etc. may be used.

As the inorganic material, monopotassium dihydrogen phosphate, dipotassium monohydrogen phosphate, magnesium sulfate, sodium chloride, ferrous sulfate, manganese sulfate, calcium carbonate, etc. may be used.

The culture liquor during the fermentation or after completion of the culturing can be used as itself. There may be used, for example, the microbial cells obtained by centrifugation of the culture liquor, washed, dried or frozen cells thereof, those treated with an organic solvent such as acetone, toluene, etc., or a surfactant, fractions containing enzymes obtained from microbial cells or treated matter thereof, immobilized microbial cells or treated matter thereof.

As the energy donor, any non-phosphorylated compound which bacteria can utilize may be used. For example, carbohydrates such as glucose, arabinose, lactose, maltose, sucrose, mannitol, solbitol, trehalose, molasses, starch hydrolyzate, etc., organic acids such as pyruvic acid, lactic acid, acetic acid and $\alpha$-ketoglutaric acid, amino acids such as glycine, alanine, aspartic acid and glutamic acid are used. These substances are used in a concentration of 1 to 200 g/l.

ATP is usually contained in microbial cells and therefore ATP sufficient for enzymatic reaction is already supplied together with the added microbial cells. However, it sometimes gives good results to add ATP or precursor thereof in a suitable amount.

In the production of glutathione, if necessary, phosphate ion and/or magnesium ion is or are added to an aqueous medium. It is preferable to maintain the concentration thereof in a range of 4-100 mM. Phosphate ion and magnesium ion may not be added provided that they are brought from the microbial cells into the reaction solution in an amount meeting the above-menitoned range of concentration.

In the production of glutathione, L-cysteine or L-cystine may be used alone or in combination thereof as the source of L-cysteine.

The enzymatic reaction to produce glutathione is carried out with stirring at 10-70 °C while adjusting the pH to a pH of 4 to 10. The addition of amino acids, organic solvents, surfactants, anti-oxidizing agents, etc. to the medium sometimes gives a good result.

Further, stirring the reaction mixture gives a good result.

Thus, the desired compound formed in the reaction mixture is recovered by conventional treatments for isolation and purification such as an ion exchange resin treatment.

Certain specific embodiments of the present invention are illustrated by the following representative examples.

## Example 1

As a microorganism, Escherichia coli ATCC 11303 was used. The strain was cultured in a medium (pH 7.2) comprising 1 g/l glucose, 10 g/l Bactotrypton (casein hydrolyzate), 5 g/l yeast extract and 5 g/l sodium chloride. The culture was inoculated on a medium (pH 7.0) comprising 10 g/l glucose, 10 g/l yeast extract, 10 g/l peptone, 5 g/l meat extract, 1 g/l $MgSO_4.7H_2O$ and 5 g/l $KH_2PO_4$ in a 2 l-Erlenmeyer flask provided with baffles. Culturing was carried out with shaking at 30 °C for one day. The culture liquor was centrifuged to obtain microbial cells, followed by freezing in a refrigerator at -20 °C.

Then 20 ml of a medium for enzymatic reaction comprising 50 g/l glucose, 1 g/l $Na_2HPO_4$, 5 g/l $MgSO_4.7H_2O$, 2.0 g/l sodium phytate, 3.5 g/l potassium sulfate, 25 mM sodium glutamate, 25 mM glycine and 25 mM L-cysteine and containing 200 g/l microbial cells (wet weight) was poured into a 200 ml-beaker and the reaction was carried out with stirring at 37 °C at a pH of 7.3 for 10 hours.

The reaction mixture was subjected to analysis by high pressure liquid chromatography and the amount of formed glutathione was 13.5 mM.

One litre of the reaction mixture obtained by the method described above was passed through a column packed with $H^+$ form of Amberlite IR-120 to adsorb glutathione on the resin. Elution was carried out with 1 N sulfuric acid to obtain an eluate, followed by concentration. The concentrate was recrystallized

from ethanol to obtain 2.7 g of purified glutathione. On the other hand, in the case where glucose was not added to the reaction solution, the amount of glutathione formed was 1.25 mM.

Example 2

The same strain as in Example 1 was cultured in the same manner as in Example 1. The microbial cells obtained by centrifugation of the culture liquor (I) or the frozen microbial cells (II) were added to the medium (A) for enzymatic reaction having the same composition as in Example 1 or the medium (B), i.e. the medium (A) supplemented by 4 g/l surfactant (Naimiin S-215, polyoxyethylene stearylamine Product of Nihonyushi Co.) and 10 ml/l xylene. The reactions were carried out under the same conditions as in Example 1 to obtain the results shown in Table 1.

Table 1

| Enzymatic reaction | Microbial cell | Medium | Glutathione (mM) |
|---|---|---|---|
| 1 | I | A | 5.03 |
| 2 | I | B | 19.5 |
| 3 | II | A | 13.5 |
| 4 | II | B | 18.6 |

Example 3

The same strain as in Example 1 was cultured in the same manner as in Example 1 to obtain microbial cells. The same enzymatic reaction as in No.4 of Example 2 was repeated except that 50, 100 or 200 g/l microbial cells by wet weight were used to obtain the results shown in Table 2.

Table 2

| Microbial cells (g/l) | Glutathione (mM) |
|---|---|
| 50 | 2.3 |
| 100 | 7.2 |
| 200 | 18.6 |

Example 4

Escherichia coli FERM BP-48 was cultured in the same manner as in Example 1. The microbial cells obtained by centrifugation of the culture liquor was added for enzymatic reaction to the medium having the same composition as in Example 1, supplemented by 80 mM sodium glutamate, 80 mM L-cysteine and the reaction was carried out under the same condition as in Example 1. The amount of glutathione formed in

the reaction solution was 50.5 mM.

Example 5

The strains indicated in Table 3 were cultured in the same manner as in Example 1 to obtain microbial cells. The same enzymatic reaction as in No. 4 of Example 2 was carried out for each strain. The results are shown in Table 3.

Table 3

| Strain | Glutathione (mM) |
|---|---|
| Enterobacter aerogenes ATCC 13048 | 6.54 |
| Proteus vulgaris FERM BP-655 | 5.16 |
| Erwinia herbicola ATCC 21434 | 5.73 |

## Claims

1. A process for the production of a given compound by the enzymatic conversion of a precursor for that compound in the presence of ATP and which comprises contacting the precursor in an aqueous culture medium containing ATP with the microbial cells, or an enzymatically active component therefrom, of a bacterium having the ability to convert the said precursor into said compound in the presence of ATP, characterised in that said microbial cells, or said enzymatically active component, are or is derived from, respectively, a bacterium having the additional ability to produce ATP in situ in said medium from an ATP precursor present in said medium and in the presence of a non-phosphorylated energy donor, such energy donor likewise being present in said medium.

2. A process according to claim 1, characterised in that said compound is glutathione, guanosine-5'-phosphate (GMP), flavin mononucleotide (FMN), $\gamma$-glutamylcysteine ($\gamma$-GC), glutamine or adenosine-triphosphate.

3. A process according to claim 2 which comprises producing glutathione enzymatically from L-glutamic acid, L-cysteine and/or L-cystine and glycine.

4. A process according to claim 1, 2 or 3, wherein said bacterium is from the genera Escherichia , Enterobacter , Proteus or Erwinia .

5. A process according to claim 4, wherein said bacterium is Escherichia coli ATCC 11303, Escherichia coli FERM BP-47, Escherichia coli FERM BP-48, Enterobacter aerogenes ATCC 13048, Proteus vulgaris FERM BP-655 or Erwinia herbicola ATCC 21434.

## Revendications

1. Procédé pour la production d'un composé donné, par conversion enzymatique d'un précurseur de ce composé, en présence d'ATP, lequel comprend la mise en contact du précurseur, dans un milieu de culture aqueux contenant de l'ATP, avec des cellules microbiennes, ou avec un composant à activité enzymatique provenant de celles-ci, d' une bactérie ayant la capacité de convertir ledit précurseur en ledit composé en présence d'ATP, caractérisé en ce que lesdites cellules microbiennes ou ledit

composant à activité enzymatique sont ou est dérivé(s), respectivement, d'une bactérie ayant la capacité supplémentaire de produire de l'ATP in situ dans ledit milieu, à partir d'un précurseur d'ATP présent dans ledit milieu et en présence d'un donneur d'énergie non phosphorylé, ce donneur d'énergie étant également présent dans ledit milieu.

**2.** Procédé selon la revendication 1, caractérisé en ce que ledit composé est le glutathion, le guanosine-5'-phosphate (GMP), le flavine-mononucléotide (FMN), la γ-glutamylcystéine (γ-GC), la glutamine ou l'adénosine-triphosphate.

**3.** Procédé selon la revendication 2, lequel comprend la production de glutathion par voie enzymatique à partir de l'acide L-glutamique, de la L-cystéine et/ou de la L-cystine et de la glycine.

**4.** Procédé selon la revendication 1, 2 ou 3, dans lequel ladite bactérie appartient aux genres Escherichia , Enterobacter , Proteus ou Erwinia .

**5.** Procédé selon la revendication 4, dans lequel ladite bactérie est Escherichia coli ATCC 11303, Escherichia coli FERM BP-47, Escherichia coli FERM BP-48, Enterobacter aerogenes ATCC 13048, Proteus vulgaris FERM BP-655 ou Erwinia herbicola ATCC 21434.

## Ansprüche

**1.** Verfahren zur Herstellung eines Stoffes durch die enzymatische Umwandlung eines Vorläufers für diesen Stoff in Gegenwart von ATP, bei dem der Vorläufer in einem ATP enthaltenden, wäßrigen Kulturmedium mit Zellen eines Bacteriums oder einem enzymatischen aktiven Bestandteil davon mit der Fähigkeit zur Umwandlung des Vorläufers in den Stoff in Gegenwart von ATP in Berührung gebracht wird, **dadurch gekennzeichnet,** daß die Zellen bzw. der enzymatisch aktive Bestandteil von einem Bacterium stammen, das die zusätzliche Fähigkeit zur in situ -Herstellung von ATP im Medium aus einem im Medium vorliegenden ATP-Vorläufer und in Gegenwart eines nicht-phosphorylierten Energiespenders besitzt, wobei dieser Energiespender ebenfalls im Medium vorliegt.

**2.** Verfahren nach Anspruch 1, **dadurch gekennzeichnet,** daß der Stoff Glutathion, Guanosin-5'-phosphat (GMP), Flavinmononucleotid (FMN, γ-Glutamylcystein (γ-GC), Glutamin oder Adenosintriphosphat ist.

**3.** Verfahren nach Anspruch 2, wobei Glutathionin enzymatisch aus L-Glutaminsäure, L-Cystein und/oder L-Cystin und Glycin hergestellt wird.

**4.** Verfahren nach Anspruch 1, 2 oder 3, wobei das Bacterium zu einer der Gattungen Escherichia , Enterobacter , Proteus oder Erwinia gehört.

**5.** Verfahren nach Anspruch 4, wobei das Bacterium Escherichia coli ATCC 11303, Escherichia coli FERM BP-47, Escherichia coli FERM BP-48, Enterobacter aerogenes ATCC 13048, Proteus vulgaris FERM BP-655 oder Erwinia herbicola ATCC 21434 ist.